# EUROPEAN PATENT APPLICATION

(11) **EP 4 650 806 A1**
(43) Date of publication of application: **19.11.2025**
(21) Application number: 25175136.8
(22) Date of filing: 08.05.2025
(51) Int. Cl.: G01R 33/34, G01R 33/3415

(54) **COIL UNIT, MAGNETIC RESONANCE IMAGING SYSTEM, AND CONTROL METHOD THEREOF**

(30) Priority: 15.05.2024 JP 2024079526
(71) Applicant: FUJIFILM Corporation, Tokyo 106-8620 (JP)
(72) Inventor: INOUE, Tomoki, Tokyo, 1068620 (JP); OCHI, Hisaaki, Tokyo, 1068620 (JP); OTAKE, Yosuke, Tokyo, 1068620 (JP)
(74) Representative: Dehns Germany Partnerschaft mbB

(57) **Abstract**

Provided are a coil unit, a magnetic resonance imaging system, and a control method of the magnetic resonance imaging system, which can be used by being adjusted according to a size of a subject. A coil unit according to the present disclosure is a coil unit that is fixed to a subject by the string-like member, and includes a plurality of receive coils that receive a nuclear magnetic resonance signal of the subject, and a coil cover that has flexibility and on which the plurality of receive coils are two-dimensionally arranged, in which the coil cover has a plurality of through-holes penetrating from one surface to the other surface, the through-holes being disposed at positions corresponding to a size of the subject, into which the string-like members are inserted.

## Description

### BACKGROUND OF THE INVENTION

### 1. Field of the Invention

The present invention relates to a coil unit, a magnetic resonance imaging system, and a control method thereof, and particularly to a technique of fixing the coil unit to a subject.

### 2. Description of the Related Art

A magnetic resonance imaging apparatus receives a nuclear magnetic resonance signal generated in a subject, and reconstructs the received signal to obtain a magnetic resonance image. In such a magnetic resonance imaging apparatus, it is necessary to fix a coil unit in which a plurality of receive coils for receiving the nuclear magnetic resonance signal are disposed, to a subject.

JP2012-130701A discloses a blanket including a first receiver coil array disposed on a first flexible substrate, in which the flexible substrate is configured to be disposed on or under a certain compartment of a patient under examination.

### SUMMARY OF THE INVENTION

The blanket disclosed in JP2012-130701A has a problem in that the size is fixed and individual adjustment cannot be made according to the size of the subject. In addition, there was room for improvement in the method of fixing the blanket to the subject.

The present invention has been made in view of such circumstances, and an object of the present invention is to provide a coil unit, a magnetic resonance imaging system, and a control method of the magnetic resonance imaging system, which can be used by being adjusted according to a size of a subject.

In order to achieve the above-described object, there is a coil unit according to a first aspect of the present disclosure, the coil unit that is fixed to a subject by a string-like member, comprising a plurality of receive coils that receive a nuclear magnetic resonance signal of the subject, and a coil cover that has flexibility and on which the plurality of receive coils are two-dimensionally arranged, in which the coil cover has a plurality of through-holes penetrating from one surface to the other surface, the through-holes being disposed at positions corresponding to a size of the subject, into which the string-like members are inserted.

According to a second aspect of the present disclosure, in the coil unit according to the first aspect, it is preferable that the plurality of through-holes are two-dimensionally arranged to correspond to positions of the plurality of receive coils.

According to a third aspect of the present disclosure, in the coil unit according to the first aspect or second aspect, it is preferable that the coil unit is fixed to the subject by the string-like member inserted into at least four through-holes.

According to a fourth aspect of the present disclosure, in the coil unit according to any one of the first to third aspects, it is preferable that the coil unit is fixed to the subject by the two string-like members.

According to a fifth aspect of the present disclosure, in the coil unit according to any one of the first to fourth aspects, it is preferable that the coil unit further comprises a detection member that detects whether or not the string-like member is inserted into each of the plurality of through-holes.

According to a sixth aspect of the present disclosure, in the coil unit according to any one of the first to fifth aspects, it is preferable that each of the plurality of through-holes includes a light emitting element that emits light in a state where the string-like member is inserted.

According to a seventh aspect of the present disclosure, in the coil unit according to any one of the first to sixth aspects, it is preferable that the coil cover is made of a transparent material.

In order to achieve the above-described object, there is a magnetic resonance imaging system according to an eighth aspect of the present disclosure comprising a string-like member, the coil unit according to any one of the first to seventh aspects, and a table on which a subject is placed, in which it is preferable that the table includes an engaging member with which the string-like member is engaged, and the subject is fixed to the table by the string-like member engaged with the engaging member.

In order to achieve the above-described object, there is a magnetic resonance imaging system according to a ninth aspect of the present disclosure comprising a string-like member, the coil unit according to any one of the first to eighth aspects and a processor that processes a nuclear magnetic resonance signal, in which the processor is configured to discriminate a plurality of through-holes into which the string-like member is inserted, among the plurality of through-holes, select a plurality of receive coils to be used based on positions of the plurality of discriminated through-holes, among the plurality of receive coils, and process the nuclear magnetic resonance signal received by the plurality of selected receive coils.

According to a tenth aspect of the present disclosure, in the magnetic resonance imaging system according to the ninth aspect, it is preferable that the processor is configured to process a nuclear magnetic resonance signal received by a receive coil disposed inside a range divided by the plurality of through-holes into which the string-like member is inserted, among the plurality of receive coils.

According to an eleventh aspect of the present disclosure, in the magnetic resonance imaging system according to the tenth aspect, it is preferable that the processor is configured to set a field of view (FOV) including an inside of the range divided by the plurality of through-holes into which the string-like member is inserted.

According to a twelfth aspect of the present disclosure, in the magnetic resonance imaging system according to any one of the ninth to eleventh aspects, it is preferable that the plurality of through-holes includes a detection member that detects whether or not the string-like member is inserted into each through-hole, and the processor is configured to discriminate the plurality of through-holes into which the string-like member is inserted, from a detection result of the detection member.

According to a thirteenth aspect of the present disclosure, in the magnetic resonance imaging system according to any one of the ninth to twelfth aspects, it is preferable that the magnetic resonance imaging system further comprises a camera, and the processor is configured to discriminate the plurality of through-holes into which the string-like member is inserted, from a detection result of the detection member.

According to a fourteenth aspect of the present disclosure, in the magnetic resonance imaging system according to the thirteenth aspect, it is preferable that each of the plurality of through-holes includes a light emitting element that emits light in a state in which the string-like member is inserted, and the processor is configured to detect the plurality of through-holes into which the string-like member is inserted, from presence or absence of the light emitted by the light emitting element.

In order to achieve the above-described object, there is a control method of a magnetic resonance imaging system according to a fifteenth aspect of the present disclosure, the magnetic resonance imaging system including a string-like member, a coil unit that is fixed to a subject by the string-like member, and includes a plurality of receive coils that receive a nuclear magnetic resonance signal of the subject, and a coil cover that has flexibility and on which the plurality of receive coils are two-dimensionally arranged, in which the coil cover has a plurality of through-holes penetrating from one surface to the other surface, the through-holes being disposed at positions corresponding to a size of the subject, into which the string-like members are inserted, and a processor, in which the processor is configured to discriminate a plurality of through-holes into which the string-like member is inserted, among the plurality of through-holes, select a plurality of receive coils to be used based on positions of the plurality of discriminated through-holes, among the plurality of receive coils and process the nuclear magnetic resonance signal received by the plurality of selected receive coils.

According to the present invention, the size of the subject can be adjusted and used.

### BRIEF DESCRIPTION OF THE DRAWINGS

Fig. 1 is a perspective view showing an example of a magnetic resonance imaging system.
Fig. 2 is a schematic diagram showing an example of an internal configuration of an MRI apparatus.
Fig. 3 is an exploded perspective view showing an example of a configuration of a coil unit.
Fig. 4 is a perspective view of the coil unit as viewed in a Y direction.
Fig. 5 is a diagram showing an example of a coil unit fixed to a subject.
Fig. 6 is a diagram showing an example of a coil unit fixed to a subject in which a size of an examination site is relatively small.
Fig. 7 is a diagram showing another example of the coil unit fixed to the subject.
Fig. 8 is a diagram showing another example of the coil unit fixed to the subject.
Fig. 9 is a diagram showing another example of the coil unit fixed to the subject.
Fig. 10 is a diagram showing an example in which the subject is fixed to a top plate by a belt.
Fig. 11 is a diagram showing an example of a configuration of a signal detection circuit in a case where the coil unit includes a switch.
Fig. 12 is a diagram showing an example of a magnetic resonance imaging system including a camera.
Fig. 13 is a perspective view showing an example of a push switch mechanism having a color developing function.
Fig. 14 is a cross-sectional view taken along line 14-14 of Fig. 13.
Fig. 15 is a diagram for describing an operation of the push switch mechanism.
Fig. 16 is a flowchart showing an example of a control method of the magnetic resonance imaging system 10.

### DESCRIPTION OF THE PREFERRED EMBODIMENTS

Hereinafter, a preferred embodiment of the present invention will be described in detail with reference to the accompanying drawings. In the following description and the accompanying drawings, the same constituent elements are denoted by the same reference numerals, and the duplicated description thereof is omitted. In addition, in the following embodiment, in a case in which a plurality of constituent elements are described and listed, it can be interpreted that at least one of the plurality of constituent elements is included.

### Magnetic Resonance Imaging System

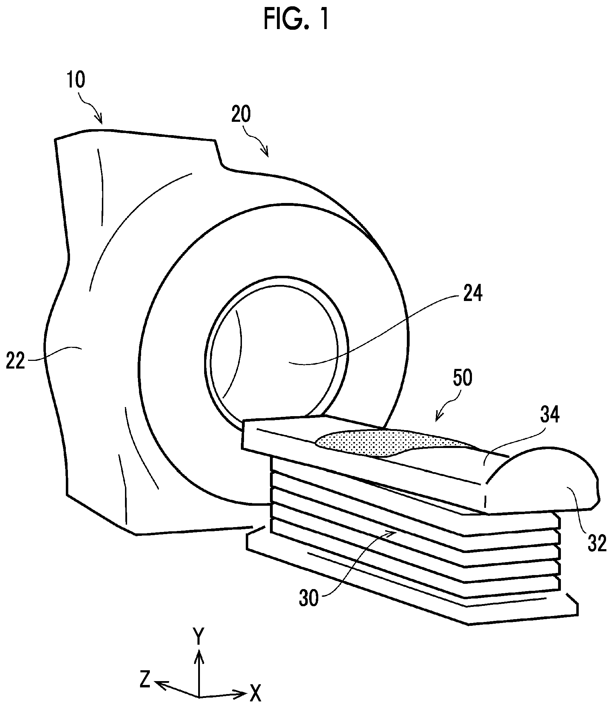

Fig. 1 is a perspective view showing an example of a magnetic resonance imaging system 10. In Fig. 1, a Z direction is a static magnetic field direction, a Y direction is a vertical direction, and an X direction is a direction orthogonal to the Y direction and the Z direction. The magnetic resonance imaging system 10 comprises a magnetic resonance imaging apparatus 20 (hereinafter, referred to as an MRI apparatus 20) and a coil unit 50.

The MRI apparatus 20 comprises a gantry 22 and a bed 30. The gantry 22 has an imaging space 24 on a cylinder. In addition, the gantry 22 has an MRI magnet and various other coils disposed therein.

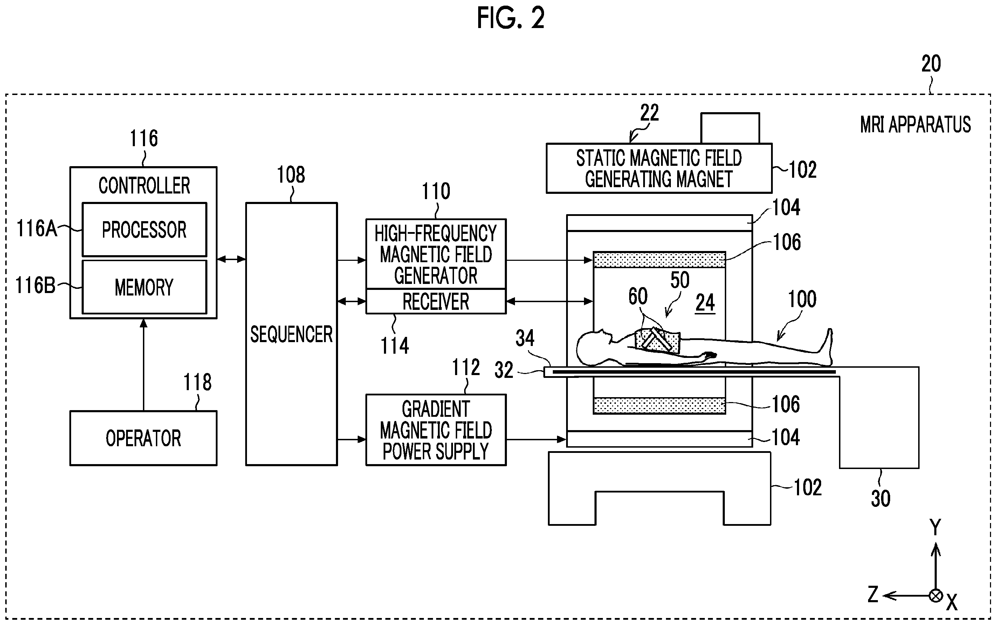

The bed 30 is installed to face the imaging space 24 on a front side of the gantry 22. The bed 30 includes a table 32. A subject 100 (see Fig. 2) is placed on a top plate 34 of the table 32. The coil unit 50 is mounted on an examination site of the subject 100. The table 32 may include a fixation mechanism for fixing the subject 100 to the top plate 34.

The bed 30 causes the table 32 to enter the imaging space 24 and exit from the imaging space 24 by a drive mechanism (not shown).

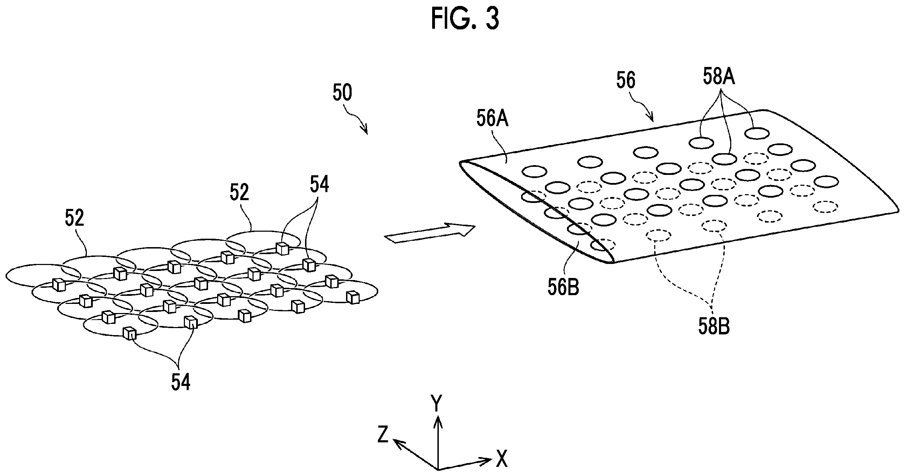

The coil unit 50 is a multi-channel radio frequency (RF) coil unit consisting of a plurality of receive coils 52 (see Fig. 3) for receiving a nuclear magnetic resonance (NMR) signal (hereinafter, referred to as an NMR signal) generated in the subject 100.

A reception-side cable (not shown) that outputs the NMR signal received by the coil unit 50 is connected to the coil unit 50. A reception-side connector (not shown) is connected to an end part of the reception-side cable. The reception-side connector is connected to a bed-side connector of a bed-side cable (not shown). The bed-side cable is connected to a controller 116 (see Fig. 2). As a result, the coil unit 50, the controller 116, and a sequencer 108 (see Fig. 2) are connected to each other in a communicable manner. The connection between the coil unit 50 and the controller 116 and the sequencer 108 is not limited to wired connection such as a cable, and an aspect in which the connection is made wirelessly is also possible.

### Internal Configuration of MRI Apparatus

Fig. 2 is a schematic diagram showing an example of an internal configuration of the MRI apparatus 20. The MRI apparatus 20 comprises a static magnetic field generating magnet 102, a gradient magnetic field coil 104, a transmit coil 106, the sequencer 108, a high-frequency magnetic field generator 110, a gradient magnetic field power supply 112, a receiver 114, the controller 116, and an operator 118.

The static magnetic field generating magnet 102 generates a uniform static magnetic field in the imaging space 24 in which the subject 100 is disposed. The gradient magnetic field coil 104 generates a gradient magnetic field in the imaging space 24. The transmit coil 106 generates a high-frequency magnetic field for causing an NMR signal to be generated in a nucleus of an atom constituting a tissue of the subject 100 in the imaging space 24.

The examination site of the subject 100 is disposed at the center of the static magnetic field of the imaging space 24. The coil unit 50 is fixed by the belt 60, which is a string-like member, to the examination site of the subject 100. The coil unit 50 detects the NMR signal generated from the subject 100.

The sequencer 108 sends commands to the high-frequency magnetic field generator 110 and the gradient magnetic field power supply 112 in accordance with the imaging sequence (pulse sequence). As a result, the signals amplified appropriately are transmitted to the transmit coil 106 and the gradient magnetic field coil 104, respectively.

The transmit coil 106 applies a pulsed high-frequency magnetic field (RF pulse) to the subject 100 in response to a signal from the high-frequency magnetic field generator 110. Preferably, the sequencer 108 sends a command to the coil unit 50 in accordance with the above-described imaging sequence and turns off the coil unit 50 during the application of the RF pulse.

The gradient magnetic field coil 104 is composed of gradient magnetic field coils in three directions of X, Y, and Z. Each of the gradient magnetic field coils 104 generates a gradient magnetic field in response to a signal from the gradient magnetic field power supply 112.

The NMR signal generated from the subject 100 is detected by the receive coil 52 of the coil unit 50, is amplified by a preamplifier (not shown) in the receive coil 52, and is transmitted to the receiver 114. In the receiver 114, the amplified NMR signal is subjected to analog-to-digital (AD) conversion and necessary signal processing to generate data. The generated data is transmitted to the controller 116. This data is also referred to as a reception signal or measurement data.

The sequencer 108 controls the operation of each unit according to pre-programmed timing and intensity. Among programs, a program that particularly describes the timing and intensity of RF pulses, gradient magnetic fields, and signal reception is referred to as a pulse sequence. Various pulse sequences depending on the purpose are known, but the detailed description thereof will be omitted here.

The controller 116 receives various instruction inputs from the operator 118 and comprehensively controls each unit of the MRI apparatus 20 via the sequencer 108. In addition, the controller 116 performs processing to convert the reception signal in the spatial frequency domain, received from the receiver 114, into an image in real space by inverse Fourier transform, and generates the MRI image.

The controller 116 is implemented using a general-purpose computer, such as a personal computer or a microcomputer. The controller 116 includes a processor 116A and a memory 116B.

The processor 116A executes an instruction stored in the memory 116B. A hardware structure of the processor 116A includes the following various processors. The various processors include a central processing unit (CPU) that is a general-purpose processor operating as various functional units by executing software (a program), a graphics processing unit (GPU) that is a processor specialized in image processing, a programmable logic device (PLD) such as a field programmable gate array (FPGA) that is a processor having a circuit configuration changeable after manufacture, a dedicated electric circuit such as an application specific integrated circuit (ASIC) that is a processor having a circuit configuration dedicatedly designed to execute specific processing, and the like.

One processing unit may be composed of one of the various processors or may be composed of two or more processors of the same type or different types (for example, a plurality of FPGAs, a combination of a CPU and an FPGA, or a combination of a CPU and a GPU). A plurality of functional units may be composed of one processor. As an example in which the plurality of functional units are configured of one processor, first, as typified by a computer such as a client or a server, one processor is configured of a combination of one or more CPUs and software and this processor acts as the plurality of functional units. Second, as typified by a system on chip (SoC) or the like, a processor that realizes the functions of the entire system including the plurality of functional units with one integrated circuit (IC) chip is used. Various functional units are configured using one or more of the various processors as a hardware structure.

In addition, the hardware structures of these various processors are more specifically electrical circuitry where circuit elements, such as semiconductor elements, are combined.

The memory 116B stores the instruction to be executed by the processor 116A. The memory 116B includes a random access memory (RAM) and a read only memory (ROM) (not illustrated). The processor 116A executes various types of processing of the MRI apparatus 20 by using the RAM as a work region, executing software by using various programs and parameters stored in the ROM, and using the parameters stored in the ROM and the like.

The controller 116 may include an input/output interface (not shown).

The operator 118 includes a mouse, a keyboard, a display, and the like. The user inputs activation, stop (including pause), selection of a pulse sequence, imaging conditions, processing conditions, and the like of the MRI apparatus 20 using the operator 118.

### Coil Unit

Fig. 3 is an exploded perspective view showing an example of a configuration of the coil unit 50. As shown in Fig. 3, the coil unit 50 includes the plurality of receive coils 52, a plurality of signal detection circuits 54, and a coil cover 56. The coil unit 50 may have the reception-side connector (not shown) for connecting the reception-side cable.

The receive coil 52 functions as a detector (sensor) that receives the NMR signal. The receive coil 52 is a circular loop coil having a diameter of approximately 10 cm to 15 cm as viewed in a Y direction. The plurality of receive coils 52 are two-dimensionally arranged on the coil cover 56. Here, the coil cover 56 incorporates a plurality of receive coils 52 arranged in a two-dimensional manner. The coil unit 50 shown in Fig. 3 comprises a total of 20 receive coils 52 that are two-dimensionally arranged in a lattice form of five rows in the X direction and four columns in the Z direction, and thus is multi-channel. Note that, the number and arrangement of the receive coils 52 are not limited to the example shown in Fig. 3. In addition, Fig. 3 shows an example of a circular shape as the shape of the receive coil, but the shape of each receive coil 52 is not limited to a circular shape, and may be an elliptical shape or a polygonal shape having a similar loop area, or a combination thereof.

The plurality of signal detection circuits 54 are disposed to correspond to each of the plurality of receive coils 52. In the signal detection circuit 54, an electric circuit including a plurality of circuit elements is packaged in a cubic or rectangular parallelepiped housing.

The coil cover 56 is a housing that covers the plurality of receive coils 52 and the plurality of signal detection circuits 54. The plurality of receive coils 52 and the plurality of signal detection circuits 54 are accommodated inside the coil cover 56 and are configured as a blanket-shaped coil unit 50. The plurality of receive coils 52 and the plurality of signal detection circuits 54 may be accommodated in the coil cover 56 in a state of being fixed to a film (not shown). The film can fix the positional relationship between the receive coil 52 and the signal detection circuit 54 and suppress the positional deviation.

The coil cover 56 has flexibility and is formed in a bag shape by sewing or adhering an end part of a sheet-like material cut into one piece. In the example shown in Fig. 3, a first sheet body 56A and a second sheet body 56B are sewn or adhered to each other to form the bag-shaped coil cover 56. The material of the coil cover 56 may be a urethane-based resin such as polyurethane, a polyamide synthetic resin such as nylon, or the like.

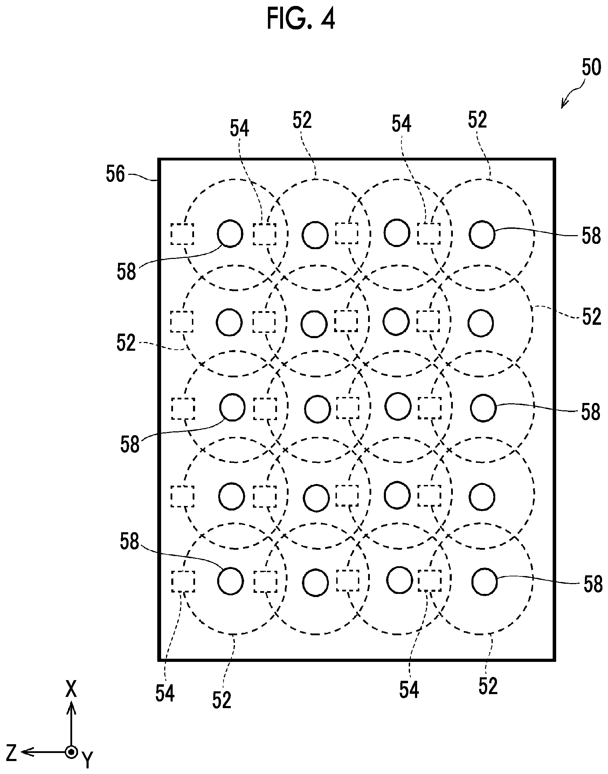

The first sheet body 56A is provided with a plurality of first holes 58A corresponding to the arrangement of the plurality of receive coils 52. The second sheet body 56B is provided with a plurality of second holes 58B corresponding to the arrangement of the plurality of receive coils 52. The first hole 58A and the second hole 58B at the corresponding positions are connected to each other by an engaging member (not shown), so that the coil unit 50 is formed with a plurality of through-holes 58 (see Fig. 4) for size adjustment that penetrate from one surface (for example, a surface formed by the first sheet body 56A) of the coil cover 56 to the other surface (for example, a surface formed by the second sheet body 56B). The size and shape of the through-hole 58 are not particularly limited, and the belt 60 may be inserted into the through-hole 58.

Note that, the coil unit 50 is connected to the controller 116 and the sequencer 108 via a cable, but the present invention is not limited thereto. The coil unit 50 and the controller 116 and the sequencer 108 may be connected wirelessly. In this case, the coil unit 50 further includes at least an analog-to-digital (AD) conversion module and a wireless communication module.

Fig. 4 is a perspective view of the coil unit 50 as viewed in the Y direction. In the example shown in Fig. 4, in the coil unit 50, a total of 20 through-holes 58, which are five rows in the X direction and four columns in the Z direction, are arranged in concentric with the loop portion of the corresponding receive coil 52. That is, the through-holes 58 are two-dimensionally arranged in a lattice shape.

Note that, the plurality of through-holes 58 may be arranged in the loop portion of one receive coil 52. The position of the through-hole 58 may be outside the loop portion of the receive coil 52.

In the following, it is assumed that the coil unit 50 fixed to the subject 100 is a surface on which the second sheet body 56B is in contact with the subject 100, and a surface on which the first sheet body 56A is not in contact with the subject 100.

### Fixing of Coil Unit

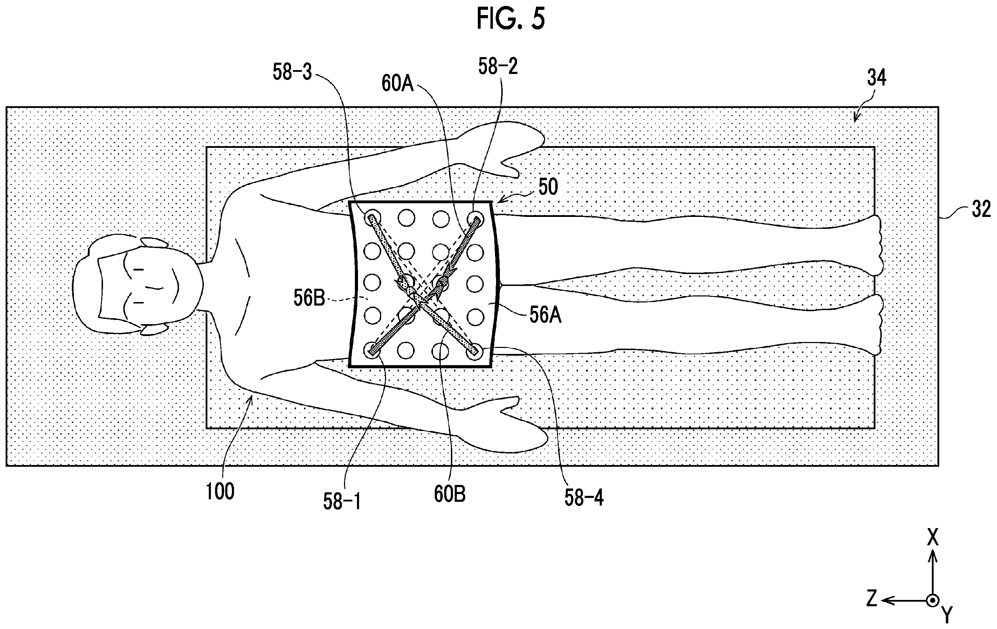

Fig. 5 is a diagram showing an example of the coil unit 50 fixed to the subject 100. In the example shown in Fig. 5, the coil unit 50 is fixed by inserting two belts 60A and 60B into four through-holes 58-1, 58-2, 58-3, and 58-4 among the plurality of through-holes 58, on the abdomen of the subject 100 placed on the top plate 34.

The belt 60A is inserted into the through-hole 58-1 of the coil unit 50 disposed on the abdomen of the subject 100 from the first sheet body 56A side to the second sheet body 56B side, is passed over the left and right sides (X direction) of the subject 100 on the back surface side of the subject 100, and is inserted into the through-hole 58-2 from the second sheet body 56B side to the first sheet body 56A side. In addition, the both ends of the belt 60A are tied at a position where the distance between the through-hole 58-1 and the through-hole 58-2 is equal on the first sheet body 56A side of the coil unit 50.

The belt 60B is inserted into the through-hole 58-3 of the coil unit 50 from the first sheet body 56A side to the second sheet body 56B side, is passed over the left and right sides of the subject 100 on the back surface side of the subject 100, and is inserted into the through-hole 58-4 from the second sheet body 56B side to the first sheet body 56A side.

In addition, the both ends of the belt 60B are tied at a position where the distance between the through-hole 58-3 and the through-hole 58-4 is equal on the first sheet body 56A side of the coil unit 50.

In the example shown in Fig. 5, the belts 60A and 60B are inserted into four through-holes 58-1, 58-2, 58-3, and 58-4 at positions corresponding to the size of the examination site of the subject 100 among the plurality of through-holes 58. Therefore, the coil unit 50 is appropriately in close contact with and fixed to the subject 100 by the belt 60A inserted into the through-holes 58-1 and 58-2 and the belt 60B inserted into the through-holes 58-3 and 58-4.

In addition, the through-holes 58-1 and 58-2 and the through-holes 58-3 and 58-4 are arranged on a diagonal line. Therefore, the belts 60A and 60B intersect each other on the back surface side and the front surface side of the subject 100. Therefore, the belts 60A and 60B can fix the coil unit 50 in close contact with the subject 100.

The coil unit 50 may be fixed by one or three or more belts 60. The belt 60 may be inserted into at least four through-holes 58, and may be inserted into five or more through-holes 58. The belt 60 may be inserted into one through-hole 58 a plurality of times or a plurality of belts 60 may be inserted into one through-hole 58.

As shown in Fig. 5, in order to fix the coil unit 50, the user first places the subject 100 on the top plate 34 of the table 32 in a supine position. In addition, the user places the coil unit 50 on the abdomen, which is the examination site of the subject 100.

Next, the user selects a plurality of through-holes 58 for fixing the coil unit 50 with the belts 60A and 60B from the plurality of through-holes 58 of the coil unit 50. For example, the user selects the four through-holes 58 disposed inside a region selected as a field of view (FOV) of the MRI apparatus 20. Here, the user further selects four through-holes 58 at positions corresponding to the size of the examination site of the subject 100. In the example shown in Fig. 5, the through-holes 58-1, 58-2, 58-3, and 58-4 are selected.

The user passes the belt 60A between the back surface of the subject 100 and the top plate 34 to the left and right sides of the subject 100. The user may pass the belt 60A to the left and right of the top plate 34 in advance before placing the subject 100 on the top plate 34 of the table 32.

Subsequently, the user passes one end of the belt 60A through the through-hole 58-1 from the second sheet body 56B side of the coil unit 50 to the first sheet body 56A side. Similarly, the user passes the other end of the belt 60A through the through-hole 58-2 from the second sheet body 56B side of the coil unit 50 to the first sheet body 56A side.

The user pulls both ends of the belt 60A to tie both ends of the belt 60A and to bundle and fix the belt 60A.

In addition, the user passes the belt 60B between the back surface of the subject 100 and the top plate 34 to the left and right sides of the subject 100. The user may pass the belt 60B to the left and right of the top plate 34 in advance before placing the subject 100 on the top plate 34 of the table 32.

Next, the user passes one end of the belt 60B through the through-hole 58-3 from the second sheet body 56B side of the coil unit 50 to the first sheet body 56A side. Similarly, the user passes the other end of the belt 60B through the through-hole 58-4 from the second sheet body 56B side of the coil unit 50 to the first sheet body 56A side.

The user pulls both ends of the belt 60B to tie both ends of the belt 60B and to bundle and fix the belt 60B.

The above procedure is an example, and the order may be changed.

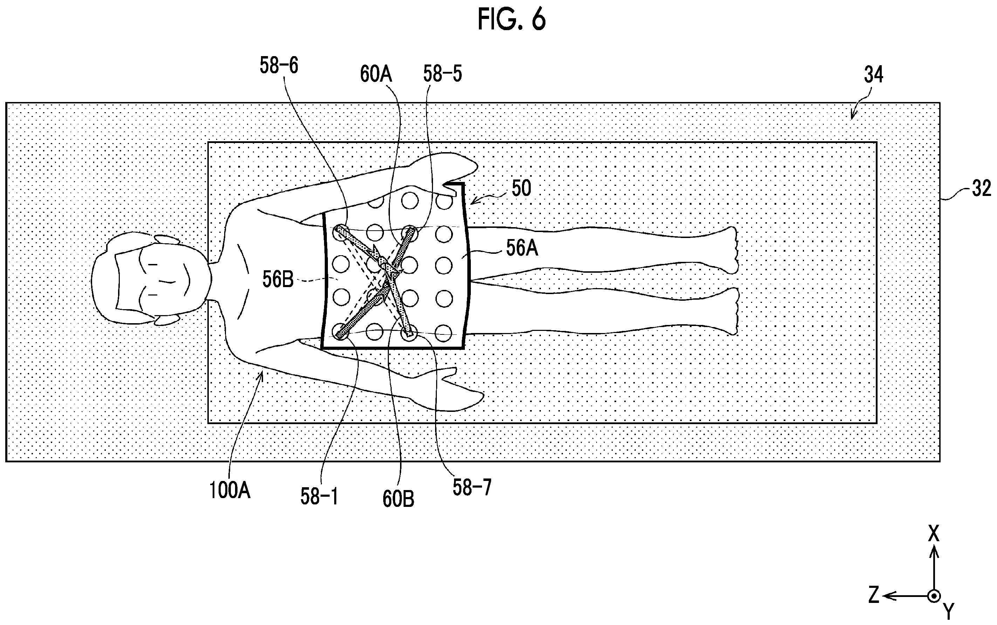

Fig. 6 is a diagram showing an example of the coil unit 50 fixed to a subject 100A in which the size of the examination site is relatively smaller than that of the subject 100. In the example shown in Fig. 6, the coil unit 50 is fixed by inserting two belts 60A and 60B into four through-holes 58-1, 58-5, 58-6, and 58-7 among the plurality of through-holes 58, on the abdomen of the subject 100A placed on the top plate 34.

The belt 60A is inserted into the through-hole 58-1 of the coil unit 50 disposed on the abdomen of the subject 100 from the first sheet body 56A side to the second sheet body 56B side, is passed over the left and right sides of the subject 100 on the back surface side of the subject 100, and is inserted into the through-hole 58-5 from the second sheet body 56B side to the first sheet body 56A side. In addition, the both ends of the belt 60A are tied at a position where the distance between the through-hole 58-1 and the through-hole 58-5 is equal on the first sheet body 56A side of the coil unit 50.

The belt 60B is inserted into the through-hole 58-6 of the coil unit 50 from the first sheet body 56A side to the second sheet body 56B side, is passed over the left and right sides of the subject 100 on the back surface side of the subject 100, and is inserted into the through-hole 58-7 from the second sheet body 56B side to the first sheet body 56A side. In addition, the both ends of the belt 60B are tied at a position where the distance between the through-hole 58-6 and the through-hole 58-7 is equal on the first sheet body 56A side of the coil unit 50.

As described above, the four through-holes 58-1, 58-5, 58-6, and 58-7 at the positions corresponding to the size of the examination site of the subject 100A are selected, and thus the coil unit 50 can be fixed in close contact with the subject 100A by the belts 60A and 60B.

The coil cover 56 may be made of a transparent material. As a result, even after the coil unit 50 is fixed to the subject 100, the user can visually recognize the body type of the subject. Therefore, the user can intuitively recognize whether or not the belt 60 is inserted into the through-hole 58 into which the belt 60 is to be inserted. The term "transparent" means that the transmittance of visible light is 50% or more, preferably 70% or more, and more preferably 90% or more.

In the examples shown in Figs. 5 and 6, the belts 60A and 60B are tied to each other to fix both ends thereof. However, the method of fixing both ends is not limited to this example. For example, a surface fastener, a band stopper, a buckle, various locking members, or the like may be used. The belts 60A and 60B may be each configured of a stretchable member.

In addition, in the examples shown in Figs. 5 and 6, the belts 60A and 60B are tied at both ends on the first sheet body 56A side (front surface side of the subject 100) of the coil unit 50, but the belts 60A and 60B may be tied at both ends on the back surface side of the subject 100.

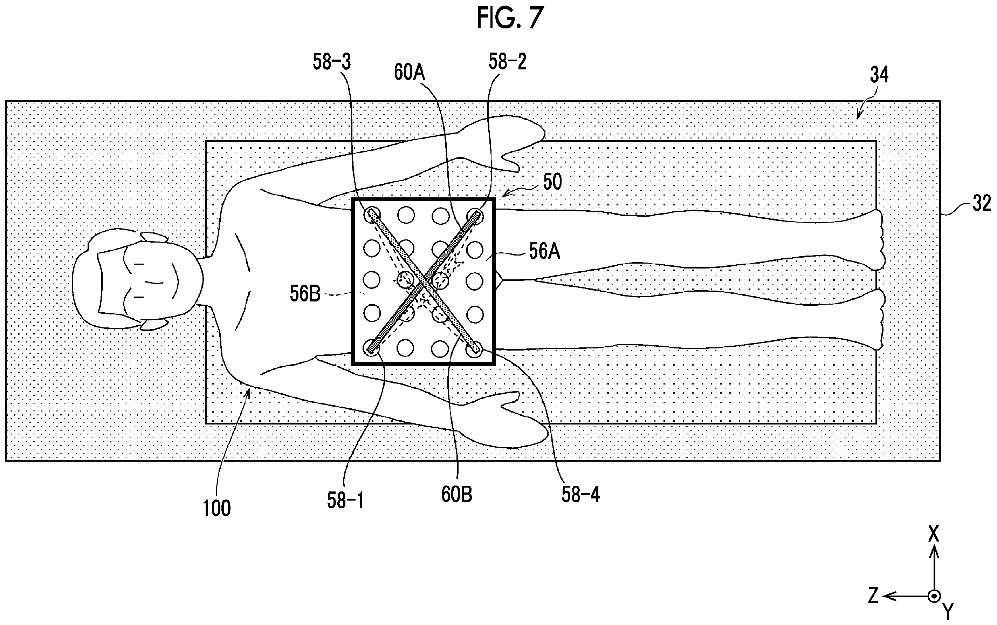

Fig. 7 is a diagram showing another example of the coil unit 50 fixed to the subject 100. As shown in Fig. 7, the belt 60A is inserted into the through-hole 58-1 of the coil unit 50 disposed on the abdomen of the subject 100 from the second sheet body 56B side to the first sheet body 56A side, and is passed over the left and right sides of the subject 100 on the first sheet body 56A side of the coil unit 50, and is inserted into the through-hole 58-2 from the first sheet body 56A side to the second sheet body 56B side. In addition, the both ends of the belt 60A are tied at a position where the distance between the through-hole 58-1 and the through-hole 58-2 is equal on the back surface side of the subject 100.

In addition, the belt 60B is inserted into the through-hole 58-3 of the coil unit 50 from the second sheet body 56B side to the first sheet body 56A side, is passed over the left and right sides of the subject 100 on the first sheet body 56A side of the coil unit 50, and is inserted into the through-hole 58-4 from the first sheet body 56A side to the second sheet body 56B side. In addition, the both ends of the belt 60B are tied at a position where the distance between the through-hole 58-3 and the through-hole 58-4 is equal on the back surface side of the subject 100.

In the case of the example shown in Fig. 7, the user may place the subject 100 on the top plate 34 after fixing the coil unit 50 to the subject 100 in advance by the belts 60A and 60B.

As described above, the coil unit 50 can be fixed in close contact with the subject 100 by the belts 60A and 60B of which both ends are tied on the back surface side of the subject 100. One of the belts 60A and 60B may be tied at both ends on the front surface side of the subject 100, and the other may be tied at both ends on the back surface side of the subject 100.

In the example shown in Figs. 5 to 7, the belts 60A and 60B intersect with each other to fix the coil unit 50, but the belts 60A and 60B may not intersect with each other.

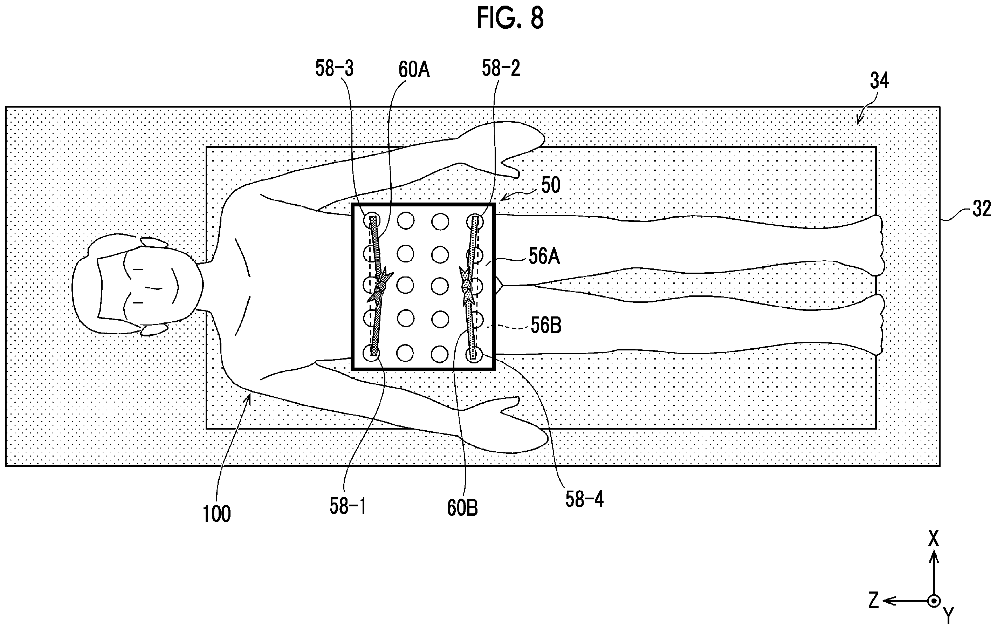

Fig. 8 is a diagram showing another example of the coil unit 50 fixed to the subject 100. As shown in Fig. 8, the belt 60A is inserted into the through-hole 58-1 of the coil unit 50 disposed on the abdomen of the subject 100 from the first sheet body 56A side to the second sheet body 56B side, is passed over the left and right sides of the subject 100 on the back surface side of the subject 100, and is inserted into the through-hole 58-3 from the second sheet body 56B side to the first sheet body 56A side. In addition, the both ends of the belt 60A are tied at a position where the distance between the through-hole 58-1 and the through-hole 58-3 is equal on the first sheet body 56A side of the coil unit 50.

In addition, the belt 60B is inserted into the through-hole 58-4 of the coil unit 50 from the first sheet body 56A side to the second sheet body 56B side, is passed over the left and right sides of the subject 100 on the back surface side of the subject 100, and is inserted into the through-hole 58-2 from the second sheet body 56B side to the first sheet body 56A side. In addition, the both ends of the belt 60B are tied at a position where the distance between the through-hole 58-2 and the through-hole 58-4 is equal on the first sheet body 56A side of the coil unit 50.

As described above, the coil unit 50 can be fixed in close contact with the subject 100 by belts 60A and 60B arranged around the subject 100 in parallel with each other.

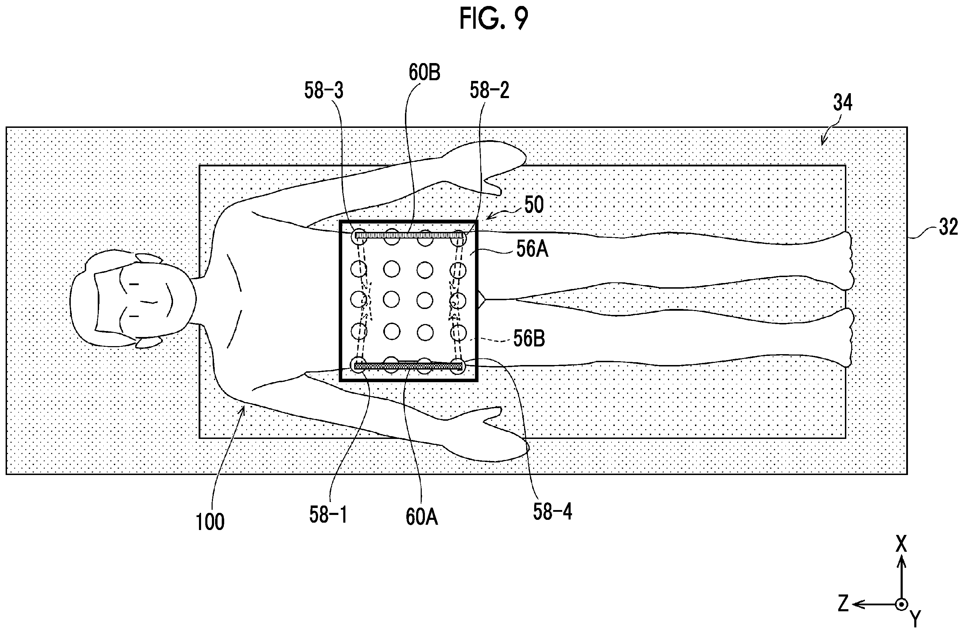

In addition, the end parts of the belts 60A and 60B may be tied. Fig. 9 is a diagram showing another example of the coil unit 50 fixed to the subject 100. As shown in Fig. 9, the belt 60A is inserted into the through-hole 58-1 of the coil unit 50 disposed on the abdomen of the subject 100 from the second sheet body 56B side to the first sheet body 56A side, is passed over the first sheet body 56A side of the coil unit 50 in the body axis direction (Z direction) of the subject 100, and is inserted into the through-hole 58-4 from the first sheet body 56A side to the second sheet body 56B side.

In addition, the belt 60B is inserted into the through-hole 58-3 of the coil unit 50 from the second sheet body 56B side to the first sheet body 56A side, is passed over the first sheet body 56A side of the coil unit 50 in the body axis direction of the subject 100, and is inserted into the through-hole 58-2 from the first sheet body 56A side to the second sheet body 56B side.

Both ends of one end of the belt 60A and one end of the belt 60B are tied at a position where the distance between the through-hole 58-1 and the through-hole 58-3 is equal on the back surface side of the subject 100. In addition, both ends of the other end of the belt 60A and the other end of the belt 60B are tied at a position where the distance between the through-hole 58-2 and the through-hole 58-4 is equal on the back surface side of the subject 100.

As described above, the coil unit 50 can be fixed in close contact with the subject 100 by the belts 60A and 60B of which the end parts are tied.

Both ends of one end of the belt 60A and the other end of the belt 60B may be tied at a position where the distance from the through-hole 58-1 and the through-hole 58-2 is equal on the back surface side of the subject 100, and both ends of the other end of the belt 60A and one end of the belt 60B may be tied at a position where the distance from the through-hole 58-3 and the through-hole 58-4 is equal on the back surface side of the subject 100. In this case, the belts 60A and 60B intersect on the back surface side of the subject 100.

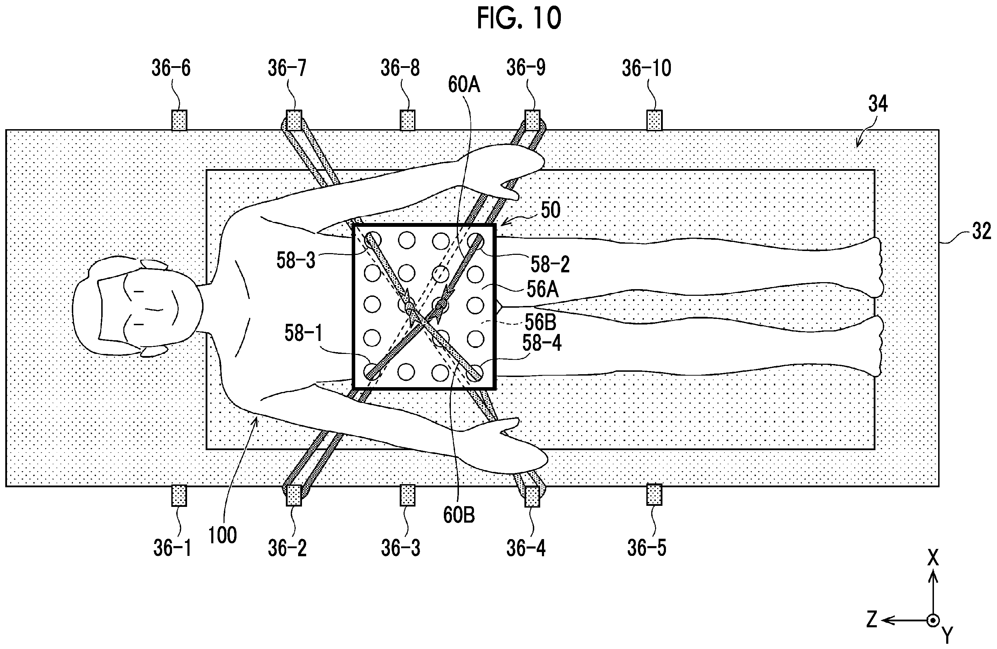

The subject 100 may be fixed to the top plate 34. Fig. 10 is a diagram showing an example in which the subject 100 is fixed to the top plate 34 by the belts 60A and 60B.

In the example shown in Fig. 10, hook members 36-1, 36-2, 36-3, 36-4, and 36-5 are provided on one end side (lower side in Fig. 10) of the table 32 in the X direction, and hook members 36-6, 36-7, 36-8, 36-9, and 36-10 are provided on the other end side (upper side in Fig. 10) of the table 32, each at regular intervals in the Z direction.

The hook members 36-1 to 36-10 are rod-shaped members of which one end is fixed to the table 32. The shapes of the hook members 36-1 to 36-10 are not limited to the rod-shaped members, and may be any shape as long as the hook members 36-1 to 36-10 can be engaged with the belts 60A and 60B. In addition, the table 32 may be provided with a hole member into which the belts 60A and 60B can be inserted, instead of the hook member.

The belt 60A is inserted into the through-hole 58-1 of the coil unit 50 disposed on the abdomen of the subject 100 from the first sheet body 56A side to the second sheet body 56B side, is engaged with the hook member 36-2, is passed over the left and right sides of the subject 100 on the back surface side of the subject 100, is engaged with the hook member 36-9, and is inserted into the through-hole 58-2 from the second sheet body 56B side to the first sheet body 56A side. In addition, the both ends of the belt 60A are tied at a position where the distance between the through-hole 58-1 and the through-hole 58-2 is equal on the first sheet body 56A side of the coil unit 50.

The belt 60B is inserted into the through-hole 58-3 of the coil unit 50 from the first sheet body 56A side to the second sheet body 56B side, is engaged with the hook member 36-7, is passed over the left and right sides of the subject 100 on the back surface side of the subject 100, and is engaged with the hook member 36-4, and is inserted into the through-hole 58-4 from the second sheet body 56B side to the first sheet body 56A side. In addition, the both ends of the belt 60B are tied at a position where the distance between the through-hole 58-3 and the through-hole 58-4 is equal on the first sheet body 56A side of the coil unit 50.

As described above, the coil unit 50 is fixed to the subject 100 by the belts 60A and 60B, and the subject 100 is fixed to the top plate 34. The user may select two of the hook members 36-1, 36-2, 36-3, 36-4, and 36-5 and two of the hook members 36-6, 36-7, 36-8, 36-9, and 36-10 to be used according to the size of the examination site of the subject 100.

### Receive Coil Used for Processing NMR Signal

The magnetic resonance imaging system 10 uses only some of the plurality of receive coils 52 provided in the coil unit 50 for processing the NMR signal. In the present embodiment, the MRI apparatus 20 selects a plurality of receive coils 52 to be used for processing the NMR signal based on the positions of the plurality of through-holes 58 into which the belt 60 is inserted.

For example, the MRI apparatus 20 processes the NMR signal received by the receive coil 52 disposed inside a range divided by the plurality of through-holes 58 into which the belt 60 is inserted among the plurality of receive coils 52. The MRI apparatus 20 may process the NMR signals received by the receive coils 52 disposed in the vicinity of a range divided by the plurality of through-holes 58 into which the belt 60 is further inserted among the plurality of receive coils 52.

The range divided by the plurality of through-holes 58 into which the belt 60 is inserted is a region surrounded by a straight line connecting the centers of the through-holes 58 into which the belt 60 is inserted. The receive coil 52 disposed inside the divided range may be a receive coil 52 in which the entire loop portion is disposed inside the divided range, or may include a receive coil 52 in which at least a part of the loop portion is disposed inside the divided range.

For example, in a case where the through-hole 58 is disposed inside the loop portion of the receive coil 52, the MRI apparatus 20 may process the NMR signal received by the receive coil 52 in which at least a part of the loop portion is disposed inside a region surrounded by a straight line connecting the centers of the through-holes 58 into which the belt 60 is inserted.

In addition, in a case where the through-hole 58 is disposed outside the loop portion of the receive coil 52, the MRI apparatus 20 may process the NMR signal received by the receive coil 52 in which the entire loop portion is disposed inside a region surrounded by a straight line connecting the centers of the through-holes 58 into which the belt 60 is inserted.

### Discrimination of Through-hole into Which Belt Is Inserted

Case Where Detection Member Is Provided in Coil Unit

The coil unit 50 may comprise a sensor or a switch that detects that the belt 60 is inserted around each through-hole 58. The sensor or the switch is connected to the signal detection circuit 54 of the corresponding receive coil 52, and the output signal of the signal detection circuit 54 is changed by the reaction of the sensor or the switch. The controller 116 discriminates the through-hole 58 into which the belt 60 is inserted by capturing this change.

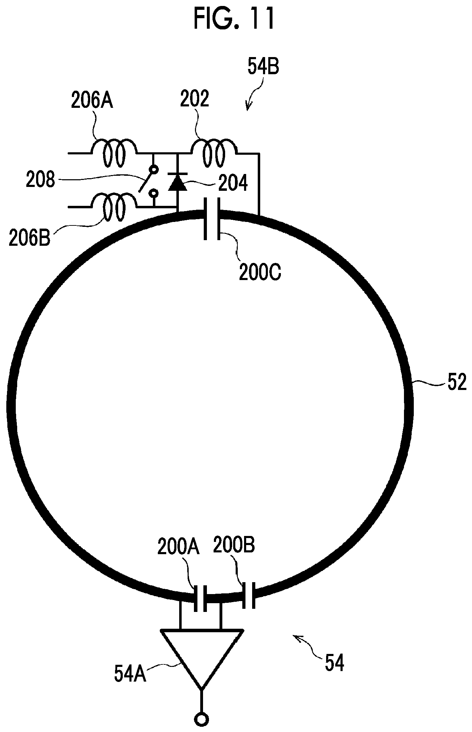

Fig. 11 is a diagram showing an example of a configuration of the receive coil 52 in a case in which the coil unit 50 comprises a switch. As shown in Fig. 11, the signal detection circuit 54 includes a preamplifier 54A, and capacitors 200A and 200B. The capacitors 200A and 200B disposed on the receive coil 52 are input impedance matching capacitors. The voltages at both ends of the capacitor 200A are input as a signal to the preamplifier 54A. In addition, the signal detection circuit 54 includes a magnetic coupling prevention circuit 54B. The magnetic coupling prevention circuit 54B prevents the magnetic coupling between the receive coil 52 and the transmit coil 106. The magnetic coupling prevention circuit 54B includes a capacitor 200C, an inductor 202, a diode 204, and choke coils 206A and 206B.

The capacitor 200C is disposed on the receive coil 52 and acts as a frequency tuning capacitor such that the resonance frequency of the receive coil 52 coincides with the magnetic resonance frequency f0 (approximately 63.88 MHz in a case of 1.5 tesla) of the MRI apparatus 20 together with the capacitors 200A and 200B. The inductor 202 and the diode 204 are connected in series to form a series circuit. The series circuit is connected in parallel to the capacitor 200C. The diode 204 is connected to a magnetic coupling prevention circuit driving device (not shown).

The choke coils 206A and 206B are connected to the anode side and the cathode side of the diode 204, respectively. The choke coils 206A and 206B prevent the alternating current from being mixed into the diode 204.

The capacitor 200C and the inductor 202 are adjusted to resonate in parallel at the magnetic resonance frequency f0 (approximately 63.88 MHz in a case of 1.5 tesla) of the MRI apparatus 20 in a case where the diode 204 is in an on state. Normally, the diode is turned on by a direct current for diode control except for a case of receiving the NMR signal. In a case where the diode is in an on state, a high impedance is generated at the position of the capacitor 200C at the magnetic resonance frequency f0, and the RF current at the magnetic resonance frequency f0 does not flow. Therefore, the receive coil 52 does not operate as a receive coil.

The magnetic coupling prevention circuit 54B further includes a switch 208 (an example of a "detection member"). The switch 208 is connected between the anode and the cathode of the diode 204. The switch 208 is in an on state in a state in which the belt 60 is inserted into the through-hole 58 (not shown in Fig. 11) corresponding to the receive coil 52 including the signal detection circuit 54, and is in an off state in a state in which the belt 60 is not inserted.

The switch 208 shorts the diode 204 in an on state. In a case where the switch 208 is in an on state, the capacitor 200C and the inductor 202 resonate at the magnetic resonance frequency f0 regardless of the on state and the off state of the diode 204, and the impedance becomes high at the position of the capacitor 200C. Therefore, the receive coil 52 does not operate as a receive coil. That is, the receive coil 52 does not resonate at the magnetic resonance frequency f0 regardless of the presence or absence of the direct current for diode control in a case where the switch 208 is in the on state. The controller 116 can discriminate the on state of the switch 208 by observing the frequency characteristic of the input impedance of the receive coil 52 (an example of the "detection result").

The controller 116 detects the through-hole 58 into which the belt 60 is inserted during so-called pre-scanning performed before the main imaging of the MRI apparatus 20. In addition, the controller 116 controls the diode 204 of the receive coil 52 located outside the range divided by the detected through-hole 58 among the plurality of receive coils 52 to an on state by the direct current for diode control. As a result, the controller 116 can select the receive coil 52 located inside the range divided by the through-hole 58 into which the belt 60 is inserted, as the receive coil 52 that receives the nuclear magnetic resonance signal.

Here, although a case where the coil unit 50 comprises a switch has been described, a transmission type laser sensor or the like can be applied as a case where the coil unit 50 comprises a sensor. For example, the light emitting element that emits laser light and the light-receiving element that receives the laser light emitted from the light emitting element may be disposed to face each other inside the through-hole 58, and the through-hole 58 into which the belt 60 is inserted may be detected by using a change in the amount of light received by the light-receiving element in a case where the belt 60 is inserted.

### Case Where Detection Member Is Provided Other Than Coil Unit

The magnetic resonance imaging system 10 may comprise a camera that images the coil unit 50 as means for detecting the through-hole 58 into which the belt 60 is inserted.

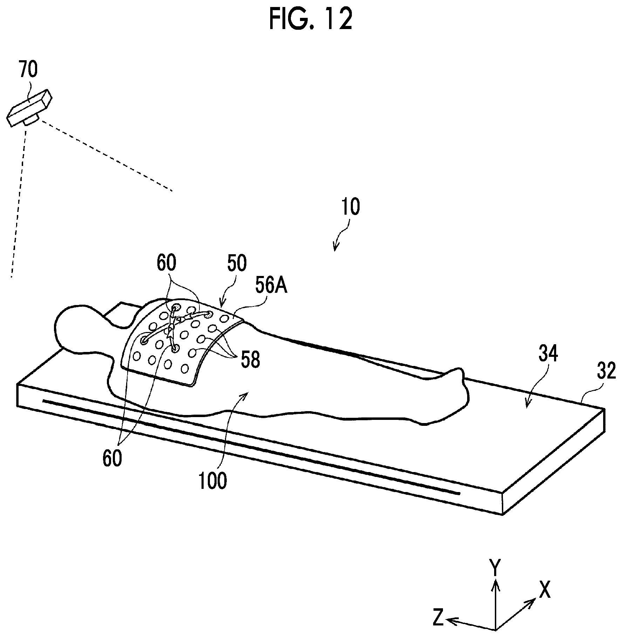

Fig. 12 is a diagram showing an example of a magnetic resonance imaging system 10 comprising a camera. As shown in Fig. 12, the camera 70 is disposed such that an optical axis is directed from above in the vertical direction toward the top plate 34 of the table 32. The camera 70 may be provided in the room of the imaging room in which the MRI apparatus 20 is installed, or may be provided inside the imaging space 24 of the gantry 22 of the MRI apparatus 20. The magnetic resonance imaging system 10 may comprise a light source that irradiates an imaging region of the camera 70 with illumination light.

A number (not shown) for identifying each through-hole 58 or a scale (not shown) for identifying the position of each through-hole 58 is given in advance by printing or the like in the vicinity of the through-hole 58 of the first sheet body 56A of the coil unit 50. The controller 116 identifies the number or the scale from the captured image of the coil unit 50 captured by the camera 70 and detects which through-hole 58 of the plurality of through-holes 58 the belt 60 is inserted into.

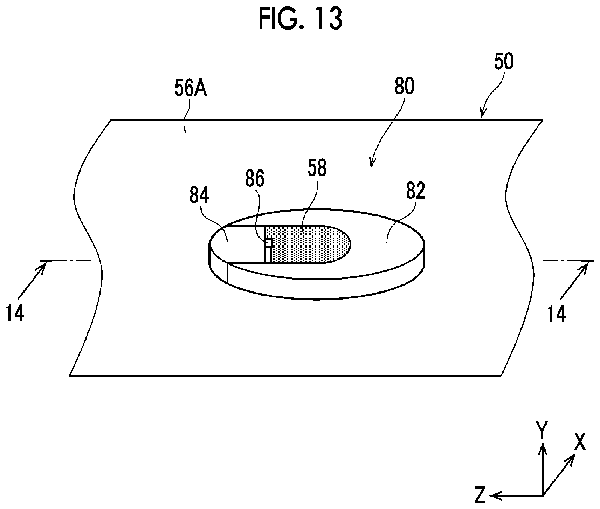

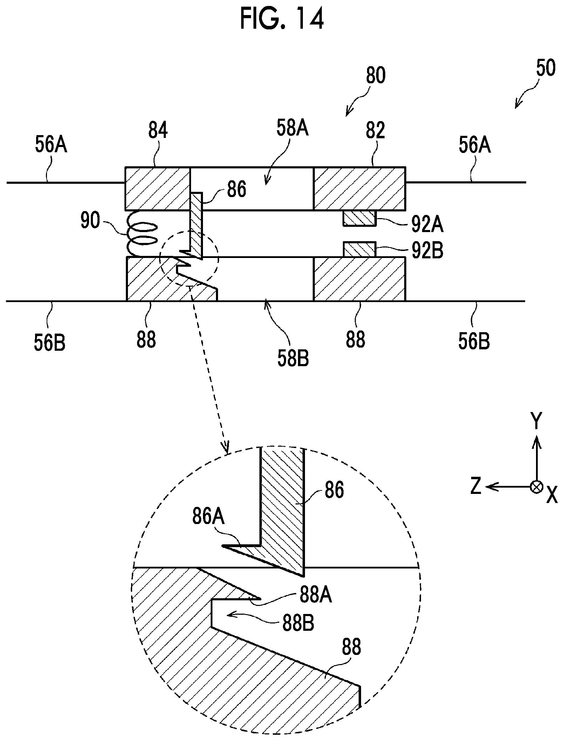

The through-hole 58 may have a mechanism that develops color or changes color in a case where the belt 60 is inserted. Fig. 13 is a perspective view showing an example of a push switch mechanism having a color developing function. The push switch mechanism 80 is provided in each of the plurality of through-holes 58, and one through-hole 58 is shown in an enlarged manner in Fig. 13. Fig. 14 is a cross-sectional view taken along line 14-14 of Fig. 13. As shown in Figs. 13 and 14, the push switch mechanism 80 includes a light emitting portion 82, a lock release button 84, a locking member 86, a ring member 88, a spring 90, a first electrode portion 92A, and a second electrode portion 92B.

The light emitting portion 82 and the lock release button 84 are disposed around the first hole 58A of the first sheet body 56A. The light emitting portion 82 is made of a transparent material and includes a light emitting element (not shown) therein. The light emitting portion 82 develops or changes color in a case where the light emitting element emits light. It is preferable that the color of the color development or the color change is a color different from the color of the coil cover 56 and the color of the belt 60. The light emitting element is driven by a charged battery (not shown). The charged battery may be charged by the direct current for diode control. The lock release button 84 is supported by the light emitting portion 82 so as to be slidably movable in a direction from the position shown in Figs. 13 and 14 toward the circumferential center of the first hole 58A.

The locking member 86 is a member that stretches in the Y direction, and is attached to be capable of being driven by the lock release button 84. A hook portion 86A that protrudes toward the outer side of the circumference of the first hole 58A is provided at a distal end of the locking member 86.

In the ring member 88, the opening portion is disposed at the position of the second hole 58B of the second sheet body 56B. The ring member 88 is provided with a protruding portion 88A that protrudes toward the circumferential center of the second hole 58B and a notchportion 88B that is recessed toward the outer side of the second hole 58B, at an inner peripheral edge portion facing the lock release button 84.

The spring 90 is an elastic member of which one end is connected to the lock release button 84 and the other end is connected to the ring member 88. The spring 90 biases the lock release button 84 in a direction (Y direction) away from the ring member 88.

The first electrode portion 92A is disposed at a position facing the ring member 88 of the light emitting portion 82, and the second electrode portion 92B is disposed at a position facing the light emitting portion 82 of the ring member 88. The light emitting portion 82 is configured such that the light emitting element emits light by the charged battery in a case where the first electrode portion 92A and the second electrode portion 92B come into contact with each other.

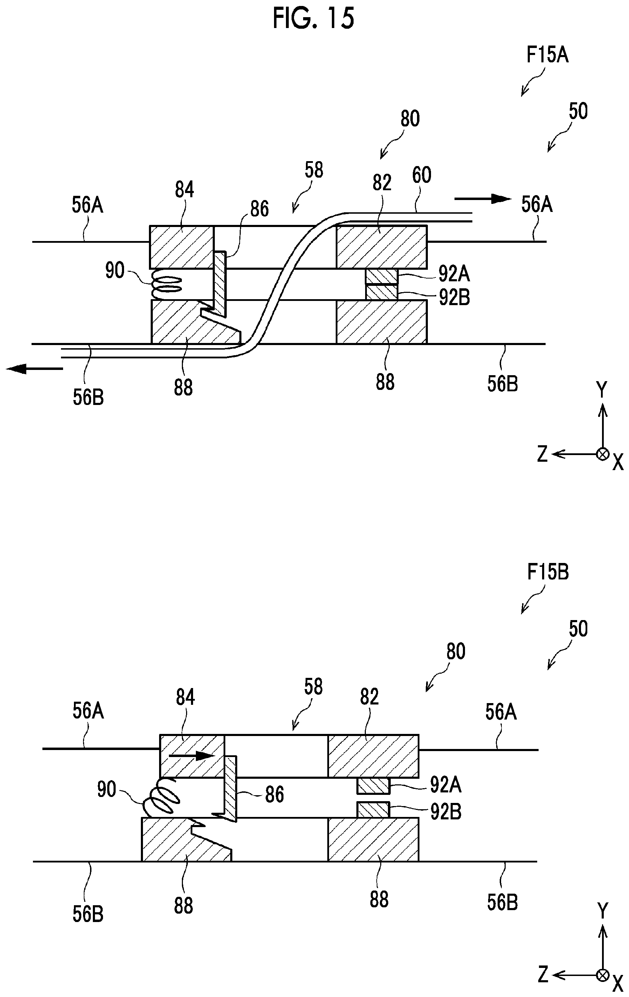

Fig. 15 is a diagram for describing the operation of the push switch mechanism 80 and is a cross-sectional view at the same position as that of Fig. 14.

In a case where the belt 60 is inserted into the through-hole 58 in the switch-off state shown in Fig. 14 and the user applies tension to the belt 60 in order to fix the coil unit 50 to the subject 100, a force is applied to the light emitting portion 82, the lock release button 84, and the ring member 88 in a direction in which the distance in the Y direction is reduced. As this force resists the biasing force of the spring 90, resulting in a switch-on state in which the first electrode portion 92A and the second electrode portion 92B are in contact with each other as shown in F15A of Fig. 15, and the light emitting element of the light emitting portion 82 emit light and the light emitting portion 82 develops or changes color. In addition, the hook portion 86A of the locking member 86 climbs over the protruding portion 88A of the ring member 88 and engages with the notch portion 88B.

Therefore, in a case where the coil unit 50 is fixed to the subject 100 by the belt 60, the light emitting portion 82 of the push switch mechanism 80 disposed in the through-hole 58 into which the belt 60 is inserted develops or changes color. As a result, the user can check the close contact between the subject 100 and the coil unit 50 with the color development or the color change at the four points. In addition, the controller 116 detects the color development or the color change of the four points from the captured image of the coil unit 50 captured by the camera 70, thereby making it easy to identify the through-hole 58 into which the belt 60 is inserted.

Even in a case where the user pulls out the belt 60 from the through-hole 58, the engagement between the hook portion 86A of the locking member 86 and the notch portion 88B of the ring member 88 is maintained, and the light emitting portion 82 maintains the color development or the color change. In order to turn off the light emitting element, as shown in F15B of Fig. 15, the user slides the lock release button 84 in a direction toward the circumferential center of the first hole 58A. Accordingly, the engagement between the hook portion 86A and the notch portion 88B is released. Therefore, the biasing force of the spring 90 causes the switch to return to the state shown in Fig. 14, the first electrode portion 92A and the second electrode portion 92B are in a non-contact switch-off state, the light emitting element of the light emitting portion 82 is turned off, and the light emitting portion 82 is in a non-color development or non-discoloration state.

The push switch mechanism 80 may be applied to the switch 208 shown in Fig. 11. In this case, the first electrode portion 92A and the second electrode portion 92B may be configured to short-circuit both ends of the diode 204 by coming into contact with each other.

### Control Method of Magnetic Resonance Imaging System

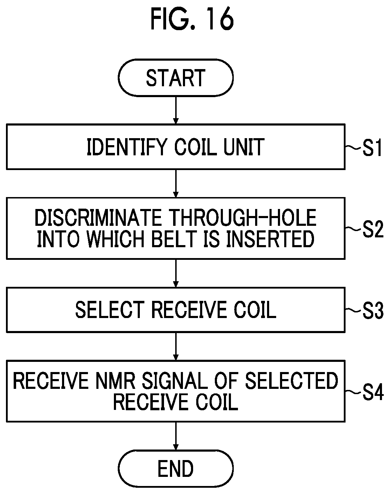

Fig. 16 is a flowchart showing an example of a control method of the magnetic resonance imaging system 10.

In step S1, the controller 116 identifies the model number and the type of the coil unit to be used. For example, the controller 116 identifies the coil unit in use by connecting the MRI apparatus 20 and the coil unit to each other in a communicable manner in a wired or wireless manner. The controller 116 recognizes the number, arrangement, size, position of through-holes, presence or absence of the detection member, and the like of the receive coils provided in the coil unit.

In addition, the user inserts the belt into the through-hole of the coil unit to fix the coil unit to the subject. Here, the user inserts the belt into the through-hole at the position corresponding to the FOV.

In step S2, the controller 116 discriminates the plurality of through-holes into which the belt is inserted, among the plurality of through-holes of the coil unit fixed to the subject. For example, in a case where the coil unit comprises the switch shown in Fig. 11, the controller 116 observes the frequency characteristics of the input impedance of the receive coil during the pre-scanning to discriminate the four through-holes into which the belt is inserted. As described with reference to Fig. 12, the controller 116 may discriminate the four through-holes into which the belt is inserted from the captured image of the coil unit captured by the camera.

In step S3, the controller 116 selects a plurality of receive coils 52 to be used based on the positions of the four through-holes discriminated in step S2, among the plurality of receive coils of the coil unit.

For example, in a case where the position of the through-hole of the coil unit is inside the loop portion of the receive coil, all the receive coils located inside the rectangle divided by the four through-holes into which the belt is inserted, including the receive coil into which the belt is inserted through the through-hole, are used. Of course, the receive coil located inside the rectangle may be used without including the receive coil into which the belt is inserted through the through-hole.

In addition, in a case where the position of the through-hole of the coil unit is outside the loop portion of the receive coil, the receive coil located inside the rectangle divided by the plurality of through-holes into which the belt is inserted, may be used. Of course, a receive coil located one row outside the receive coil located inside the rectangle may also be used in the meaning of the margin.

In addition, the controller 116 sets the FOV in a range including the plurality of receive coils 52 to be used. Accordingly, it is possible to shorten the time for the user to select the FOV.

In step S4, the controller 116 receives the NMR signals received by the plurality of receive coils selected in step S3, and reconstructs the received NMR signals by performing signal processing on the received NMR signals to acquire a magnetic resonance image.

The controller 116 may activate only the receive coil selected in step S3 to be capable of receiving the NMR signal. This can be realized by causing the direct current for diode control to flow through the magnetic coupling prevention circuit 54B of the receive coil excluding the receive coil selected in step S3. As described in Fig. 11, the receive coil in which the direct current for diode control is allowed to flow to turn on the diode 204 does not operate as the receive coil. Therefore, as a result, only the receive coil selected in step S3 can be activated to be capable of receiving the signal. The controller 116 may limit the receive coil that transmits the data to the MRI apparatus 20 by activating all the receive coils to the receive coil selected in step S3. The controller 116 may transmit the reception data of all the receive coils to the MRI apparatus 20 and limit the reception data of the receive coil used for image generation to the reception data of the receive coil selected in step S3.

### Others

The technical scope of the present invention is not limited to the scope described in the above-described embodiments. The configurations and the like in each embodiment can be appropriately combined between each of the embodiments without departing from the gist of the present invention.

### Explanation of References

50: coil unit
52: receive coil
54: signal detection circuit
56: coil cover
56A: first sheet body
56B: second sheet body
58: through-hole
60A: belt
60B: belt
70: camera
80: push switch mechanism
100: subject

## Claims

1. A coil unit that is fixed to a subject by a string-like member, the coil unit comprising:
a plurality of receive coils that receive a nuclear magnetic resonance signal of the subject; and
a coil cover that has flexibility and on which the plurality of receive coils are two-dimensionally arranged,
wherein the coil cover has a plurality of through-holes penetrating from one surface to the other surface, the through-holes being disposed at positions corresponding to a size of the subject, into which the string-like members are inserted.

2. The coil unit according to claim 1,
wherein the plurality of through-holes are two-dimensionally arranged to correspond to positions of the plurality of receive coils.

3. The coil unit according to claim 1 or 2,
wherein the coil unit is fixed to the subject by the string-like member inserted into at least four through-holes.

4. The coil unit according to any one of claims 1 to 3,
wherein the coil unit is fixed to the subject by the two string-like members.

5. The coil unit according to any one of claims 1 to 4, further comprising:
a detection member that detects whether or not the string-like member is inserted into each of the plurality of through-holes.

6. The coil unit according to any one of claims 1 to 5,
wherein each of the plurality of through-holes includes a light emitting element that emits light in a state where the string-like member is inserted.

7. The coil unit according to any one of claims 1 to 6,
wherein the coil cover is made of a transparent material.

8. A magnetic resonance imaging system comprising:
a string-like member;
the coil unit according to any one of claims 1 to 7; and
a table on which a subject is placed,
wherein the table includes an engaging member with which the string-like member is engaged, and
the subject is fixed to the table by the string-like member engaged with the engaging member.

9. A magnetic resonance imaging system comprising:
a string-like member;
the coil unit according to any one of claims 1 to 7; and
a processor that processes a nuclear magnetic resonance signal,
wherein the processor is configured to:
discriminate a plurality of through-holes into which the string-like member is inserted, among the plurality of through-holes;
select a plurality of receive coils to be used based on positions of the plurality of discriminated through-holes, among the plurality of receive coils; and
process the nuclear magnetic resonance signal received by the plurality of selected receive coils.

10. The magnetic resonance imaging system according to claim 9,
wherein the processor is configured to process a nuclear magnetic resonance signal received by a receive coil disposed inside a range divided by the plurality of through-holes into which the string-like member is inserted, among the plurality of receive coils.

11. The magnetic resonance imaging system according to claim 10,
wherein the processor is configured to set a field of view (FOV) including an inside of the range divided by the plurality of through-holes into which the string-like member is inserted.

12. The magnetic resonance imaging system according to any one of claims 9 to 11,
wherein the plurality of through-holes include a detection member that detects whether or not the string-like member is inserted into each through-hole, and
the processor is configured to discriminate the plurality of through-holes into which the string-like member is inserted, from a detection result of the detection member.

13. The magnetic resonance imaging system according to any one of claims 9 to 12, further comprising:
a camera,
wherein the processor is configured to discriminate the plurality of through-holes into which the string-like member is inserted, from an image obtained by imaging the coil unit with the camera.

14. The magnetic resonance imaging system according to claim 13,
wherein each of the plurality of through-holes include a light emitting element that emits light in a state in which the string-like member is inserted, and
the processor is configured to detect the plurality of through-holes into which the string-like member is inserted, from presence or absence of the light emitted by the light emitting element.

15. A control method of a magnetic resonance imaging system, the magnetic resonance imaging system including a string-like member, a coil unit that is fixed to a subject by the string-like member, and includes a plurality of receive coils that receive a nuclear magnetic resonance signal of the subject, and a coil cover that has flexibility and on which the plurality of receive coils are two-dimensionally arranged, in which the coil cover has a plurality of through-holes penetrating from one surface to the other surface, the through-holes being disposed at positions corresponding to a size of the subject, into which the string-like members are inserted, and a processor,
wherein the processor is configured to:
discriminate a plurality of through-holes into which the string-like member is inserted, among the plurality of through-holes;
select a plurality of receive coils to be used based on positions of the plurality of discriminated through-holes, among the plurality of receive coils; and
process the nuclear magnetic resonance signal received by the plurality of selected receive coils.
